# EUROPEAN PATENT SPECIFICATION

(11) **EP 2 932 967 B1**
(45) Date of publication and mention of the grant of the patent: **29.08.2018**
(21) Application number: 13855732.7
(22) Date of filing: 11.11.2013
(51) Int. Cl.: A61K 31/409, A61P 17/00, A61K 31/197, A61K 31/221

(54) **USE OF A PHOTOSENSITIVE AGENT CAPABLE OF PRODUCING REACTIVE OXYGEN SPECIES IN THE PRODUCTION OF A DRUG FOR THE PHOTODYNAMIC THERAPY OF A DISEASE RELATED TO STEM CELLS, IN VITRO USE, AND PHARMACEUTICAL COMPOSITION**
VERWENDUNG EINES LICHTEMPFINDLICHEN MITTELS ZUR HERSTELLUNG EINER REAKTIVEN SAUERSTOFFSPEZIES BEI DER HERSTELLUNG EINES ARZNEIMITTELS ZUR PHOTODYNAMISCHEN THERAPIE EINER KRANKHEIT IM ZUSAMMENHANG MIT STAMMZELLEN, DESSEN IN-VITRO-VERWENDUNG UND PHARMAZEUTISCHE ZUSAMMENSETZUNG
UTILISATION D'UN AGENT PHOTOSENSIBLE POUVANT PRODUIRE DES ESPÈCES RÉACTIVES D'OXYGÈNE DANS LA PRÉPARATION D'UN MÉDICAMENT UTILE POUR LA THÉRAPIE PHOTODYNAMIQUE D'UNE MALADIE ASSOCIÉE AUX CELLULES MÈRES, UTILISATION "IN VITRO" ET COMPOSITION PHARMACEUTIQUE

(30) Priority: 14.11.2012 ES 201231759
(43) Date of publication of application: 21.10.2015
(73) Proprietor: Universidad Autónoma de Madrid, 28049 Madrid (ES); Consejo Superior de Investigaciones Cientificas, 28006 Madrid (ES)
(72) Inventor: ESPADA REGALADO, Jesús, E-28029 Madrid (ES); CARRASCO CERRO, Elisa, E-28029 Madrid (ES); CALVO SANCHEZ, María Inmaculada, E-Madrid 28029 (ES); BLAZQUEZ-CASTRO, Alfonso, E-28029 Madrid (ES); JUARRANZ DE LA FUENTE, Angeles, E-28049 Madrid (ES)
(74) Representative: Hoffmann Eitle
(86) International application number: PCT/ES2013/070779
(87) International publication number: WO 2014/076338

(56) References cited:
- WO-A1-03/039597
- WO-A1-2004/093993
- WO-A1-2010/015087
- WO-A1-2011/107478
- WO-A2-2012/040105
- US-A1- 2004 029 856
- US-A1- 2010 222 538
- FERNANDEZ-GUARINO M ET AL: "Photodynamic Therapy: New Indications", ACTAS DERMOSIFILIOGRAFICAS (ENGLISH EDITION), ELSEVIER, AMSTERDAM, NL, vol. 98, no. 6, 1 January 2007 (2007-01-01), pages 377-395, XP026952162, ISSN: 1578-2190 [retrieved on 2007-01-01]
- YUKI MOROKUMA ET AL: "Hair growth stimulatory effect by a combination of 5-aminolevulinic acid and iron ion", INTERNATIONAL JOURNAL OF DERMATOLOGY, vol. 47, no. 12, 1 December 2008 (2008-12-01), pages 1298-1303, XP55241808, UK ISSN: 0011-9059, DOI: 10.1111/j.1365-4632.2008.03783.x
- ALFONSO BLÁZQUEZ-CASTRO ET AL: "Protoporphyrin IX-dependent photodynamic production of endogenous ROS stimulates cell proliferation", EUROPEAN JOURNAL OF CELL BIOLOGY, vol. 91, no. 3, 1 March 2012 (2012-03-01), pages 216-223, XP55247370, DE ISSN: 0171-9335, DOI: 10.1016/j.ejcb.2011.12.001
- EWAN A. LANGAN ET AL: "Mind the (Gender) Gap: Does Prolactin Exert Gender and/or Site-Specific Effects on the Human Hair Follicle?", JOURNAL OF INVESTIGATIVE DERMATOLOGY, vol. 130, no. 3, 1 March 2010 (2010-03-01) , pages 886-891, XP55247487, US ISSN: 0022-202X, DOI: 10.1038/jid.2009.340
- HAMANAKA R B ET AL: "Mitochondrial reactive oxygen species regulate cellular signaling and dictate biological outcomes", TRENDS IN BIOCHEMICAL SCIENCES, ELSEVIER, HAYWARDS, GB, vol. 35, no. 9, 1 September 2010 (2010-09-01), pages 505-513, XP027249508, ISSN: 0968-0004 [retrieved on 2010-04-27]
- BLANPAIN CÉDRIC ET AL: "Epidermal homeostasis: a balancing act of stem cells in the skin", NATURE REVIEWS. MOLECULAR CELL BIOLOGY ENGLAND MAR 2009,, vol. 10, no. 3, 1 March 2009 (2009-03-01), pages 207-217, XP009141979,
- CHOONG M L ET AL: "A novel role for proliferin-2 in the ex vivo expansion of hematopoietic stem cells", FEBS LETTERS, ELSEVIER, AMSTERDAM, NL, vol. 550, no. 1-3, 28 August 2003 (2003-08-28), pages 155-162, XP004448385, ISSN: 0014-5793, DOI: 10.1016/S0014-5793(03)00844-5
- R. B. HAMANAKA ET AL: "Mitochondrial Reactive Oxygen Species Promote Epidermal Differentiation and Hair Follicle Development", SCIENCE SIGNALING, vol. 6, no. 261, 5 February 2013 (2013-02-05), pages ra8-ra8, XP55247429, US ISSN: 1945-0877, DOI: 10.1126/scisignal.2003638
- ELISA CARRASCO ET AL: "Photoactivation of ROS Production In Situ Transiently Activates Cell Proliferation in Mouse Skin and in the Hair Follicle Stem Cell Niche Promoting Hair Growth and Wound Healing", JOURNAL OF INVESTIGATIVE DERMATOLOGY, vol. 135, no. 11, 1 November 2015 (2015-11-01), pages 2611-2622, XP055248026, US ISSN: 0022-202X, DOI: 10.1038/jid.2015.248
- SARDINA. J. L.: 'REACTIVE OXYGEN SPECIES: ARE THEY IMPORTANT FOR HAEMATOPOIESIS? CRITICAL REVIEWS IN ONCOLOGY/HEMATOLOGY' CRITICAL REVIEWS IN ONCOLOGY / HEMATOLOGY - ELSEVIER SCIENCE IRELAND LTD., LIMERICK, IE vol. 81, no. 3, March 2012, pages 257 - 274, XP028890992
- ZHANG. L.. ET: 'AN UPDATED VIEW ON STEM CELL DIFFERENTIATION INTO SMOOTH MUSCLE CE LL S. VASCULAR PHARMACOLOGY' vol. 56, no. 5-6, May 2012, pages 280 - 287, XP055218035
- JI A-R: 'REACTIVE OXYGEN SPECIES ENHANCE DIFFERENTIATION OF HUMAN EMBRYONIC STEM CELLS I.NCO RNESENCLOCLERMAL LINEAGE.' EXPERIMENTAL & MOLECULAR MEDICINE vol. 42, no. 3, 31 March 2010, pages 175 - 186, XP055218039
- RAO. J.: 'PHOTODYNAMIC THERAPY FOR THE DERMATOLOGIST', [Online] 01 June 2012, XP008177679 Retrieved from the Internet: <URL:<URL:HTTP://EMEDICINE.MEDSCAPE.COM/ART ICLE/ 112 .1517-OVERVIEW>.>

## Description

### FIELD OF THE INVENTION

The present invention relates to the use of protoporhyrin IX, or a precursor thereof, capable of producing reactive oxygen species, for the *in vitro* activation of epidermal stem cells.

### BACKGROUND

Photodynamic therapy (PDT) is a therapeutic modality widely used in clinical practice for the treatment of different skin pathologies, including cancer.

PDT is based on the exogenous administration of photosensitive (PS) compounds or precursors thereof which accumulate by means of different mechanisms preferably in the target tissues. Irradiating the tissue with light having a suitable wavelength, generally in the red region of the spectrum (λ>600 nm) for greater tissue penetration, and in the presence of intracellular oxygen, induces the production of reactive oxygen species (ROS), particularly singlet oxygen. The rapid intracellular accumulation of ROS above a critical threshold promotes a strong photosensitization which induces cell death.

5-aminolevulinic acid (ALA), and to a greater extent its methylated derivative, methyl aminolevulinate (MAL), are two of the most widely used compounds in clinical practice in dermatological protocols with PDT. Their low molecular weight determines a high absorption through the epidermis, allowing topical application thereof. These compounds are not photoactive themselves, but act as precursors of endogenous PS, protoporphyrin IX (PpIX). Once absorbed by the cell, they are incorporated in the heme group metabolic biosynthetic pathway, promoting an abnormal accumulation of PpIX which can last between hours and days, with the subsequent photosensitization of the target tissue. PDT-MAL treatment is extremely widespread in clinical dermatology, particularly for the treatment of actinic keratosis and basal cell carcinoma.

It has been described that experimental treatment with a low amount of exogenous ROS sources, such as hydrogen peroxide, can promote cell proliferation in *in vitro* cultures (Boonstra J, Post JA. "Molecular events associated with reactive oxygen species and cell cycle progression in mammalian cells". Gene 337:1-13, 2004), including potential neural progenitor cells grown by means of the neurosphere system (Le Belle JE et al. "Proliferative neural stem cells have high endogenous ROS levels that regulate self-renewal and neurogenesis in a PI3K/Akt-dependant manner", Cell Stem Cell. 8:59-71, 2011).

However, so far there has been no experimental evidence indicating a causal relationship between endogenous ROS production in a tissue and functional activation of a type of stem cell contained in said tissue involving physiological consequences, with potential clinical, pharmacological or cosmetic use. This is largely due to the non-existence of an "*in vivo*" experimental method which allows inducing controlled endogenous ROS production in a tissue.

There is also no experimental data indicating that endogenous ROS accumulation in tissues may be part of a normal homeostatic process that is functionally dependent on stem cells. In contrast, all the experimental results showing an "*in vivo*" ROS accumulation in tissues indicate that this accumulation is abnormal and is associated with pathological conditions and aging processes (Valko M et al. "Free radicals and antioxidants in normal physiological functions and human disease", Int. J. Biochem. Cell Biol. 39: 44-84, 2007). Likewise, topical treatment of a tissue with exogenous ROS sources cannot be considered to be biologically equivalent to physiological endogenous ROS production in any case.

The inventors describe an experimental method using PDT-MAL to induce endogenous ROS production in the hair follicle capable of activating the epidermal stem cells contained in this niche. This epidermal stem cell stimulation is due to the transcriptional activation of genes of the prolactin 2 family, also known as proliferins, particularly proliferin-2 or Prl2c3, in the target tissue by ROS. Taking into account that a potential role has been proposed for Prl2c3 in the *in vitro* expansion of hematopoietic stem cells (Choong ML et al. "A novel role for proliferin-2 in the ex vivo expansion of hematopoietic stem cells", FEBS Lett. 550:155-62, 2003), the "*in vivo*" stimulation of genes of the proliferin family by ROS associated with epidermal stem cell activation is a surprising and important discovery in its own right.

In the current art, one of the most widespread uses of epidermal stem cells in the area of bioengineering is the generation of skin equivalents having an epidermal or dermal-epidermal component (Shevchenko RV et al. "A review of tissue-engineered skin bioconstructs available for skin reconstruction", J. R. Soc. Interface 7:229-58, 2010). These skin equivalents or artificial skins have very important applications in regenerative medicine, fundamentally for the treatment of widely extended and deep burns and wounds. The ideal treatment for injuries of this type is an autograft with skin equivalents of different types generated from the patient's own skin. Given the restrictions established in the European Union (Directive 2010/63/EU) and other countries for the use of experimental animals, another fundamental application of skin equivalents is the use thereof as biological models to test the viability and toxicity of pharmaceutical and cosmetic compounds.

An essential limitation in applications of this type is the time for generating a functional skin equivalent, which requires the "*ex vivo*" expansion of epidermal progenitors and stratification of the epidermal component in contact with air. In a clinical setting, excessively long times in the production of equivalents for autograft entail an immediate risk for the patient, making the use of alternative therapies such as cadaver skin grafts or non-human synthetic equivalents necessary. In pharmacology and cosmetics, the time for generating artificial skin is directly related to its production cost. Therefore, one problem that arises in the art concerns the development of an experimental method for speeding up these processes during the formation of the skin equivalent.

Therefore, the problem that arises in the art concerns the development of methods that are more specific and effective than current methods for generating skin equivalents. The solution provided by the present invention is the use of protoporhyrin IX, or a precursor thereof, capable of producing reactive oxygen species, for the *in vitro* activation of epidermal stem cells and thus for the production of skin equivalents..

### DESCRIPTION

The present invention relates to the use of the photosensitive agent protoporphyrin IX (PpIX), or the precursor thereof, capable of producing ROS for the "in vitro" activation of stem cells, and another additional embodiment is the use thereof for producing prolactin family 2 proteins "in vitro", even more preferably proliferin-2.

The term "photosensitive agent" is understood herein as a compound which is capable of producing ROS in the presence of oxygen upon being irradiated with light having a suitable wavelength and is administered exogenously or produced by the body itself from a precursor.

The term "precursor of a photosensitive agent" is understood herein as a compound which is administered exogenously and gives rise to a photosensitive compound in the cell.

The term "reactive oxygen species" or ROS is understood herein as compounds having free radicals and ions of oxygen and peroxides.

The term "photodynamic therapy" or PDT is understood herein as a method through which ROS production in a target tissue is induced after promoting the accumulation of a photosensitive agent in said tissue and irradiating it in the presence of oxygen with light having a suitable wavelength.

In a preferred embodiment, said precursor of a photosensitive agent is a precursor of PpIX, even more preferably 5-aminolevulinic acid or the chemical derivatives thereof, and the most preferable among these chemical derivatives is methyl aminolevulinate (MAL).

The term "chemical derivative" of 5-aminolevulinic acid (ALA) is understood herein an organic compound containing the basic chemical structure of ALA having one or more chemical substituents or radicals in one of the atoms of said basic structure.

The term "stem cell activation" is understood herein as the stimulation of stem cell proliferation and/or functional differentiation programs.

The invention describes a PDT-adapted methodology which allows generating a controlled, endogenous ROS production specifically in the prominent region of the hair follicle. This results in the activation of the proliferation of the epidermal stem cells contained in this niche, acceleration of hair growth, an increase of dermal collagen and induction of the expression of genes of prolactin family 2 (proliferins), particularly Prl2c3.

The stimulation of stem cells according to the present invention has a potential direct application in the area of bioengineering for speeding up the growth of skin equivalents having an epidermal or dermal-epidermal component (artificial skins) used in regenerative medicine and as substrates to test the biological viability of pharmacological and cosmetic compounds. Therefore, a technological embodiment of the invention is the use of a photosensitive agent, or the precursor thereof, capable of producing ROS for generating dermal-epidermal or epidermal skin equivalents.

Another preferred embodiment is the photosensitive agent of the invention protoporphyrin IX (PpIX), or the precursor thereof, capable of producing ROS, for the "*in vitro*" activation of stem cells, and another additional preferred embodiment is that it is for producing prolactin family 2 proteins, even more preferably proliferin-2, "*in vitro*"*.*

In this sense, yet another additional embodiment is the photosensitive agent, or the precursor thereof, capable of producing ROS, of the invention, for generating dermal-epidermal or epidermal skin equivalents.

### BRIEF DESCRIPTION OF THE DRAWINGS

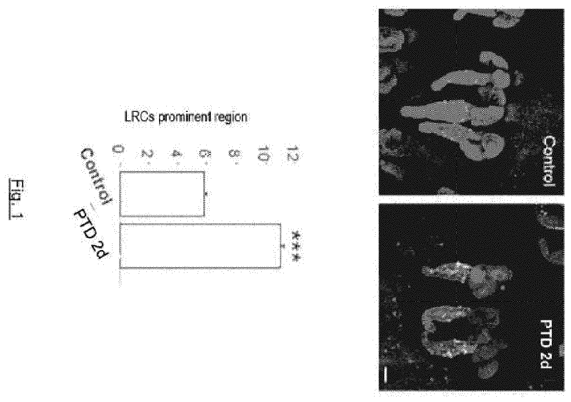

Figure 1 shows that PDT induces cell proliferation in the prominent region of the hair follicle.

The generated labeled cells (Label Retaining Cells, LRCs) were detected by means of the administration of serial injections of thymidine analog, BrdU, to newborn mice and further analysis at 50 days, and quantified by immunofluorescence in *"in toto"* mounts of adult mice tail epidermis. A significant increase in LRCs was observed in the prominent region of the hair follicle (HF) in telogen two days after treatment (PDT 2d) with respect to controls, which indicates stimulation of the proliferation of the HF epidermal stem cells in response to treatment. The bars indicate the standard error. Scale bar: 100 µm. ***: Significant, P<0.001.

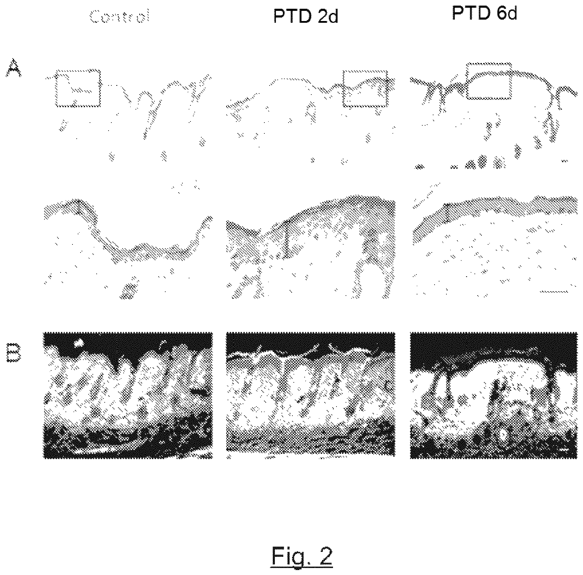

Figure 2 shows that PDT induces transient morphological changes in the skin.

A: In the histological sections of the dorsal skin of the back, a transient hyperplasia was observed in the epidermis two days after photodynamic stimulation, and it regressed six days after said stimulation. The regions highlighted in the upper panels are shown in detail in the lower panels.

B: The fluorescence of eosin shows an increase in the density of collagen fibers of the dermis observed 6 days after PDT. Staining: H-E. Scale bar: 20 µm.

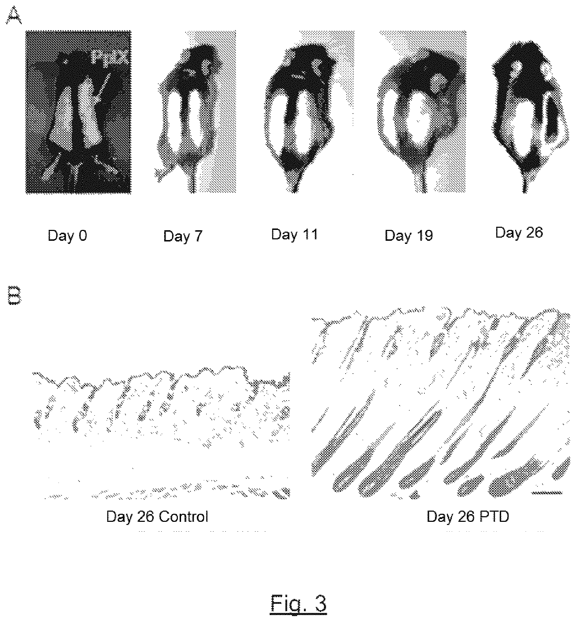

Figure 3 shows that PDT induces hair growth.

A: Two separate areas of the skin of the back of the mice were shaved and Metvix® was applied topically on the right region, while the left region, used as a control, was left untreated. PpIX production in the region treated with Metvix® (day 0) was checked by means of red fluorescence emission under excitation with UV light. After PDT, accelerated hair growth was observed in the treated skin region with respect to the control (days 7 to 26).

B: The H-E-stained histological sections of dorsal skin samples obtained 26 days after treatment showed the progress of the anagen phase of the HFs in the skin treated with PDT. Scale bar: 100 µm.

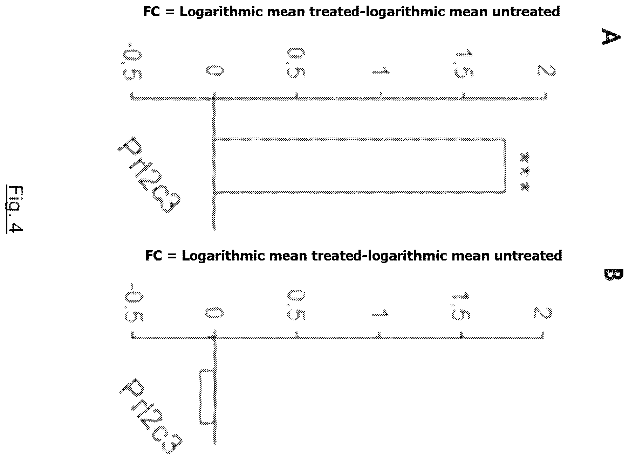

Figure 4 shows that PDT causes changes in skin gene expression.

A: Real-time quantitative PCR analysis based on skin samples from the back of treated mice and their corresponding controls showed the induction of Prl2c3 mRNA which encodes proliferin-2, two days after applying PDT-MAL.

B: No significant changes were observed in the expression of Prl2c3 mRNA six days after treatment. 18S ribosomal RNA was used as endogenous RNA for normalization and the relative quantification (RQ) was calculated based on the control. ***: Significant, P<0.001.

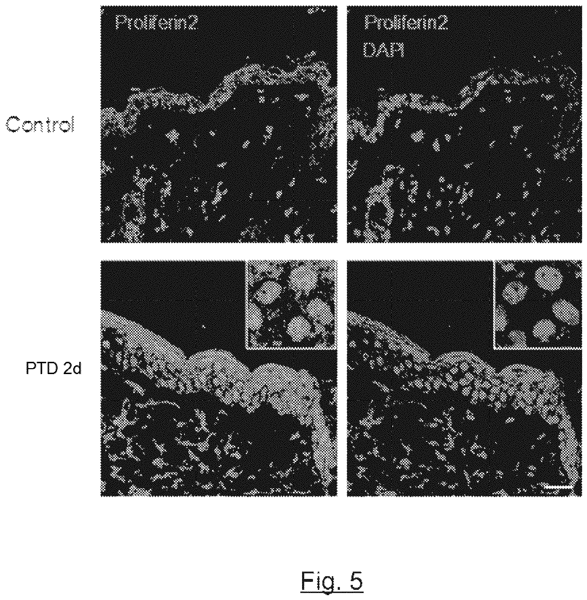

Figure 5 shows that PDT causes changes in the expression and localization of proliferin-2 in the skin. The immunofluorescence in histological skin sections showed an increased expression of proliferin-2 in the epidermis after applying PDT-MAL and a novel nuclear localization of this protein as a result of the treatment. Scale bar: 50 µm.

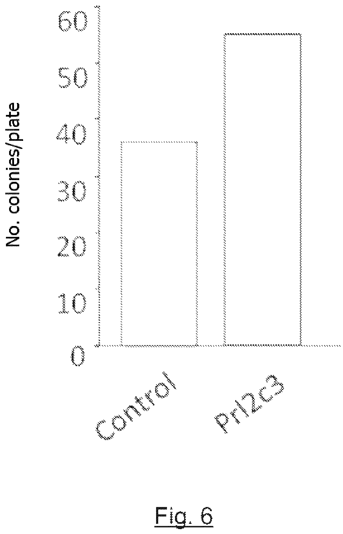

Figure 6 shows the induction of "*in vitro*" epidermal stem cell growth and expansion by Prlc3. Epidermal stem cells were isolated from mouse skin and grown in a conditioned culture medium containing high amounts of Prl2c3 or in a control medium. The number of colonies with at least four cells, indicating sustained proliferative activity, was quantified in these cultures after seven days. The results indicated a significant increase in the number of colonies in the epidermal stem cell cultures treated with Prl2c3-conditioned growth medium.

### PREFERRED EMBODIMENTS

The following examples are provided to demonstrate the present invention in an illustrative but non-limiting manner.

### Example 1: Experimental animals

Ten-day old newborn mice from the C57BL/6 line were used for the epidermal stem cell labeling experiments, and 7-week old adult mice were used for the rest of the tests. The animals used in each experiment were littermates and the comparisons were established between individuals of the same sex to avoid differences attributable to this factor. The experiments were performed in accordance with the regulations governing the handling and care of laboratory animals (Royal Decree 1201/2005).

### Example 2: Photodynamic therapy application

The skin of the back of the mice was shaved and shaving cream (Veet®) was applied. The methylated derivative of ALA (MAL) was administered the next day on the skin of the back and the tail in the form of a commercial cream (Metvix®, Galderma) and incubated in the dark for 5 hours, after which excess Metvix® was removed by washing with PBS. Endogenous PpIX production in the skin of the back was checked using red fluorescence emission characteristic of PpIX under excitation with a 407 nm UV light, using a digital chamber provided with two lamps of said wavelength. The animals were then anesthetized by means of intraperitoneal (i.p.) injection of a 3:1 solution of Imalgene 500 (Merial) and Domtor (Pfizer) (50 µl/mouse; 0.864 mg of ketamine hydrochloride and 0.005 mg of medetomidine hydrochloride per 10 grams of body weight). Irradiation with a 636 nm red light was carried out evenly over the dorsal surface of the tail and the back for 3.5 minutes using a 36 J/cm² diode lamp (Aktilite®) located 5 cm from the animal. After exposure to the red light, the animals received a subcutaneous injection of 2:1 Antisedan (Pfizer) with respect to the volume of Domtor administered, and were kept on thermal blankets until complete recovery. After the time determined for each test elapsed, the animals were sacrificed in a CO₂ chamber and the skin was processed for different analyses.

In the case of functional capillary induction experiments, the skin of the back was first shaved in two separate areas and Metvix® was applied only in the right region, keeping the left half as a control.

### Example 3: Labeling of epidermal stem cells identified as Label Retaining Cells (LRCs)

The epidermal stem cells were labeled and identified according to the Braun protocol (Braun et al, "Manipulation of stem cell proliferation and lineage commitment: visualization of label-retaining cells in wholemounts of mouse epidermis". Development. 130:5241-55, 2003). The newborn mice received an i.p. injection of 50 mg/kg of body weight of BrdU (Sigma-Aldrich) (80 µl of 6.25 mg/ml BrdU) in PBS once a day for four consecutive days, so that the DNA in all the skin cells can be extensively labeled. After 7 weeks, the epidermal stem cells were identified based on the low replication rate that characterizes them as cells capable of retaining the BrdU label (Label Retaining Cells, LRCs) for a prolonged time period due to the sporadic replication of their DNA. The identification and quantification of LRCs after PDT-MAL treatment was carried out by means of immunofluorescence in "*in toto*" mounts of tail epidermis that were observed under a confocal microscope.

### Example 4: Processing of the skin, histology and immunofluorescence

Immediately after sacrificing the animal, the tail was separated from the body and an incision was made with a scalpel in the ventral area thereof, the skin being separated by hand in a single piece. The skin was incubated in 10 ml of 5 mM EDTA in PBS for 6 hours at 37°C and the epidermis was then separated from the dermis with the help of forceps. The epidermis samples were transversely divided into two portions: one was frozen at -80°C for RNA extraction and the other was fixed in 3.7% formaldehyde in PBS for 48 hours at 4°C, they were washed in PBS and stored in 0.02% PBS-sodium azide for the preparation of "*in toto*" mounts.

On the other hand, the skin of the back was removed and fixed in 3.7% formaldehyde in PBS for at least 48 hours at 4°C. They were then embedded in paraffin following standard protocols and 4 µm histological sections that were stained with hematoxylin-eosin (HE) or processed for immunofluorescence (IF) were prepared, using in this last case poly L-lysine-treated slides. For IF tests, the deparaffinized and hydrated sections were soaked in 0.1% Triton X-100 in PBS, autofluorescence was removed by means of incubation with 50 mM NH₄Cl (10 minutes at room temperature), and they were blocked in 0.3% bovine serum albumin (BSA, Sigma) in PBS (1 hour at room temperature). The blocked samples were incubated overnight at 4°C with a polyclonal antibody (Santa Cruz Biotechnology) that is specific against prolactin family 2 proteins (proliferins), including proliferin-2 (Prl2c3). They were then washed in PBS and incubated with the corresponding Cy3-coupled secondary antibody (Jackson ImmunoResearch Laboratories). Finally, the samples were washed in PBS and mounted with Vectashield (Vector Labs) containing 5 ng/ml of DAPI (Merck). For the detection of LRCs by means of immunofluorescence in "*in toto*" mounts, the epidermis pieces of the tail were incubated with 1 N HCl (45 minutes at 37°C) and with Tris-borate-EDTA (5 minutes at room temperature), performing respective brief washes with distilled water after each incubation. They were then soaked and blocked in a PTG buffer (0.5% Triton X-100, 0.2% gelatin in PBS) for 1 hour at room temperature, after which they were incubated with the fluorescein isothiocyanate (FITC)-conjugated mouse anti-BrdU primary monoclonal antibody (Roche) overnight at 37°C. They were then washed repeatedly in PBS and mounted with Vectashield-DAPI. The immunofluorescence samples were analyzed under a spectral confocal microscope, Leica TCS-SP2-AOBS, using excitation lasers of 488 nm for FITC, 633 nm for Cy3 and UV for DAPI. Three-dimensional reconstructions were performed with the help of LCS Suite version 2.61 (Leica) and then processed with Photoshop CS3 Extended version 10.0.1 (Adobe). The histological sections stained with HE were analyzed under an Olympus BX61 fluorescence microscope coupled to an Olympus DP50 digital camera, using clear field and blue excitation light (excitation filter BP 460-490 and barrier filter BA 520IF).

### Example 5: Prl2c3 RNA extraction, large-scale analysis of gene expression patterns and qRT-PCR

The purification of Prl2c3 RNA from the epidermis of the tail and the skin of the back was performed by means of organic extraction with TriPureTM Isolation Reagent (Roche) followed by column purification (RNeasy Mini kit, QIAGEN). The tissue was broken up with a pair of scissors and homogenized in TriPure with a Polytron (PT 1200 E, Kinematica). The homogenate was phase separated in chloroform:isoamyl (Merck) and the RNA of the aqueous phase was column purified. The concentration and purity of the RNA (A260:A280 ratio > 1.8) were determined by means of spectrophotometry (Nanodrop ND1000, Nanodrop Technologies). The large-scale analysis of gene expression was performed by means of arrays of Agilent Technologies (Agilent.SingleColor. 14868). The real-time quantitative PCR analyses (qRT-PCR) of the expression were carried out using 7900HT Fast Real Time PCR with SYBR Green of Applied Biosystems.

### Results:

### Example 6: Induction of the proliferation of the stem cells found in the prominent region of the hair follicle by means of PDT-MAL treatment

To analyze the effect of PDT-MAL treatment on the activity of epidermal stem cells, the hair follicle (HF), the prominent region of which constitutes the main reservoir of skin stem cells, was used as a model. The stem cells were identified based on their characteristic low proliferation rate which allows retaining a nuclear BrdU label for a prolonged time period after the serial administration of the nucleotide analog in newborns, which allows identifying them as LRCs (Braun et al, "Manipulation of stem cell proliferation and lineage commitment: visualization of label-retaining cells in wholemounts of mouse epidermis". Development. 130:5241-55, 2003; Cotsarelis et al, "Label-retaining cells reside in the bulge area of pilosebaceous unit: implications for follicular stem cells, hair cycle, and skin carcinogenesis". Cell 61, 1329-37, 1990). As shown in Figure 1, the immunofluorescence analysis in "*in toto*" mounts of the epidermis of the tail of control mice and mice subjected to PDT-MAL showed a significant increase (P<0.001) in the number of LRCs in the prominent region of the HFs.

### Example 7: Induction of transient hyperplasia in the epidermis and increase in the density of collagen fibers in the dermis by means of PDT-MAL treatment

The large-scale morphological changes caused by PDT-MAL were characterized in histological sections of the dorsal skin of the back stained with HE. The analysis of these sections indicated that the treatment induces transient hyperplasia in the epidermis, the most obvious response being observed 2 days after treatment, and regressing to normal state 7 days after treatment (Figure 2A). Very pronounced changes were also observed in the morphology of the dermis which showed a significant increase in the density of collagen fibers 7 days after MAL-PDT treatment, characterized as an increase in the specific fluorescent emission of eosin as it weaves itself into collagen fibers (Espada et al. "Selective fluorescence of eosinophilic structures in grasshopper and mammalian testis stained with haematoxylin-eosin" Histochemistry 99:385-390, 1993) (Figure 2B).

### Example 8: Hair growth acceleration by means of PDT-MAL treatment

To analyze the dynamics of hair growth in the skin subjected to PDT-MAL, the skin of the back of the mice was shaved and Metvix® was applied topically on the right dorsal region, keeping the left as a control, as described in detail in Example 2. After 5 hours of incubation in the dark elapsed, production was determined PpIX by means of analyzing the red fluorescent emission characteristics of this compound. The obtained results showed that PpIX production from MAL in the epidermis took place in the presence of Metvix®, as indicated by the fluorescent signal observed under UV excitation light (Figure 3A, day 0). PDT was then completed irradiating the back of the mice with red light. The progression observed in the subsequent days showed a significantly accelerated hair growth in the half treated with respect to the control skin (Figure 3A, days 7 to 26). The histological sections corresponding to skin samples taken 26 days after PDT clearly show the development achieved by HFs (Figure 3B). Therefore, while HFs in the control skin remain at rest (telogen) and in no case exceed the dermis, the progress of the growth phase (anagen) is observed in the treated skin and the hair bulb region penetrates extensively into the layer underlying the dermis.

### Example 9: Specific induction of the Prl2c3 gene by means of PDT-MAL treatment

To determine the changes in the gene expression pattern induced in the skin by means of PDT-MAL treatment, a large-scale microarray RNA analysis was carried out using mRNA obtained from the skin of the back and the epidermis of the tail. By means of this approach, the product of the Prl2c3 gene was identified as the mRNA the expression of which was seen to be strongly modified in response to PDT-MAL treatment, result which was validated by means of qRT-PCR (Figure 4). The immunolocalization of protein Prl2c3 in histological dorsal skin sections showed a low intensity and diffuse pattern in cytoplasm in the epidermis of control animals, while the increase in the expression levels after treatment was confirmed, disclosing a novel nuclear localization of this protein in animals subjected to PDT-MAL (Figure 5).

### Example 10: Induction of the "in vitro" growth and expansion of epidermal stem cells by means of a culture medium containing high amounts of Prl2c3

Prl2c3 cDNA was first isolated by means of RT-PCR then cloned into expression vector pcDNA3.1A, and this vector was transfected into HEK293T cells. It was verified by means of immunoblotting that the culture medium conditioned by the growth of cells transfected with the cloned vector contained, on average, up to 10 times more Prlc3 protein than a control medium containing cells transfected with the empty vector. Epidermal stem cells were then isolated from mouse skin according to established protocols (Espada et al. "Nuclear envelope defects cause stem cell dysfunction in premature-aging mice" J. Cell Biol. 181:27, 35, 2008). These cultures were treated with a conditioned medium. The cultures with conditioned medium showed a significantly larger number of colonies with more than 4 cells compared to control cultures treated with medium containing cells transfected with the empty vector (Figure 6).

## Claims

1. Use of protoporhyrin IX, or a precursor thereof, capable of producing reactive oxygen species, for the *in vitro* activation of epidermal stem cells.

2. Use of protoporhyrin IX, or a precursor thereof, capable of producing reactive oxygen species, for the *in vitro* production of proteins of the prolactin family 2.

3. The use according to claim 2, wherein the proteins of the prolactin family 2 are proliferin-2.

4. Use of protoporhyrin IX, or a precursor thereof, capable of producing reactive oxygen species, for the generation of dermo-epidermal or epidermal skin equivalents.

5. The use according to any of claims 1 to 4, wherein the precursor is 5-aminolevulinic acid.

## Patentansprüche

1. Verwendung von Protoporphyrin IX, oder einer Vorstufe desselben, welches in der Lage ist, reaktive Sauerstoffspezies für die In-vitro-Aktivierung von epidermalen Stammzellen herzustellen.

2. Verwendung von Protoporphyrin IX, oder einer Vorstufe desselben, welches in der Lage ist, reaktive Sauerstoffspezies für die In-vitro-Herstellung von Proteinen der Prolaktin-Familie 2 herzustellen.

3. Verwendung nach Anspruch 2, wobei die Proteine der Prolaktin-Familie 2 Proliferin-2 sind.

4. Verwendung von Protoporphyrin IX, oder einer Vorstufe desselben, welches in der Lage ist, reaktive Sauerstoffspezies für die Erzeugung von dermoepidermalen oder epidermalen Hautäquivalenten herzustellen.

5. Verwendung nach einem der Ansprüche 1 bis 4, wobei die Vorstufe 5-Aminolävulinsäure ist.

## Revendications

1. Utilisation de protoporphyrine IX, ou un précurseur de celle-ci, apte à produire des espèces réactives d'oxygène, pour l'activation *in vitro* de cellules souches épidermiques.

2. Utilisation de protoporphyrine IX, ou un précurseur de celle-ci, apte à produire des espèces réactives d'oxygène, pour la production *in vitro* de protéines de la famille de prolactine 2.

3. Utilisation selon la revendication 2, dans laquelle les protéines de la famille de prolactine 2 sont de la proliférine-2.

4. Utilisation de protoporphyrine IX, ou un précurseur de celle-ci, apte à produire des espèces réactives d'oxygène, pour la génération d'équivalents dermo-épidermique ou de peau d'épiderme.

5. Utilisation selon l'une quelconque des revendications 1 à 4, dans laquelle le précurseur est de l'acide 5-aminolévulinique.
